# EUROPÄISCHE PATENTANMELDUNG

(11) **EP 4 559 490 A1**
(43) Veröffentlichungstag der Anmeldung: **28.05.2025**
(21) Anmeldenummer: 24214042.4
(22) Anmeldetag: 19.11.2024
(51) Int. Cl.: A61M 1/14

(54) **ZENTRIERENDE SCHNITTSTELLE**

(30) Priorität: 21.11.2023 DE 102023132347
(71) Anmelder: B. Braun Avitum AG, 34212 Melsungen (DE)
(72) Erfinder: KNIERIM, Michael, 37242 Bad Sooden-Allendorf (DE)
(74) Vertreter: Winter, Brandl - Partnerschaft mbB

(57) **Zusammenfassung**

Beschrieben wird eine zentrierende Schnittstelle zwischen einem in einer Vorrichtung montierten rohrförmigen Element, insbesondere einem Aufnahmerohr oder Köcher (34), für einen Ansaugstab eines Dialysegeräts, und einem vorzugsweise schwenkbar um eine Achse geführten beweglichen Element, insbesondere einer Frontplatte (24) des Dialysegeräts (20). Es ist eine Zentrierflächenpaarung (42, 50) zwischen in der Vorrichtung montiertem Element (34) und beweglichem Element (24) vorgesehen, und bei der Herstellung einer vorzugsweise abgedichteten Fügung der Schnittstelle ist das in der Vorrichtung montierte Element (34) bei der Bewegung des beweglichen Elements (24) und der damit einhergehenden Annäherung der Zentrierflächen (42, 50) in axialer Richtung gegen die Kraft einer Vorspannfeder bewegbar, während seine führende Lagerung eine Bewegung zulässt, die es erlaubt, die Zentrierflächen (42, 50) zwängungsfrei in Fügeposition mit zueinander fluchtenden Zentrierflächenachsen (A42, A50) zu bringen.

## Beschreibung

Die Erfindung betrifft eine zentrierende Schnittstelle zwischen einem vorrichtungsfest montierten rohrförmigen Element, insbesondere einem Aufnahmerohr (Köcher) für einen Ansaugstab eines Dialysegeräts, und einem schwenkbar um eine Achse geführten beweglichen Element, insbesondere einer Frontplatte eines Dialysegeräts.

Solche Schnittstellen werden beispielsweise bei medizintechnischen Geräten, wie Dialysegeräten, benötigt, um eine saubere Schnittstelle für einen Ansaugstab zu schaffen, mit dem Bicarbonat und/oder Konzentrat aus in der Regel auf einem Sockel positionierten Kanistern entnommen werden kann. Am Dialysegerät sind hierzu in der Regel sogenannte Köcher zur Aufnahme nicht verwendeter oder für den Spülvorgang gelagerter Ansaugstäbe vorgesehen.

Es gibt bekannte einschlägige Schnittstellen, bei denen sich beispielsweise zwei vertikal übereinanderliegende Köcher im Dialysegerät befinden, während in einer schwenkbeweglichen Frontplatte des Dialysegeräts formgenaue Durchbrüche zur Aufnahme der stirnseitigen Enden der Köcher ausgebildet sind. Durch die Toleranzkette ist es aber selbst mit manueller Einstellung, die einen erheblichen zeitlichen Montageaufwand nach sich zieht, nur schlecht möglich, eine saubere Schnittstelle zu erreichen. Das Resultat ist ein für den Anwender unerwünschter sichtbarer Spalt, wodurch Flüssigkeit in die Maschine gelangen kann und schlechte Reinigung möglich ist.

Um die Entstehung unerwünschter Spalte zu vermeiden, wird der Köcher in anderen bekannten Geräten als eine einzelne feststehende Baugruppe ausgebildet, die keine beweglichen Bauteile mehr besitzt.

Da die Anwender zwischenzeitlich erwarten, dass sich alle Anschlüsse und Schnittstellen bequem zugänglich und ergonomisch optimiert an der beweglichen Frontplatte befinden, und weil unterhalb der Frontplatte in der Regel kein Raum für eine feststehende Köcher-Baugruppe mehr verbleibt, weil dort Kanister für Konzentrat und dgl. stehen, besteht ein Bedürfnis, eine Schnittstelle der eingangs beschriebenen Art zu schaffen, die es erlaubt, den in dem Dialysegerät montierten Köcher beim Schließen der Frontplatte sauber und spaltfrei abzudichten, ohne dass aufwendige Montage- und Justierhandgriffe erforderlich werden.

Diesem Bedürfnis wird durch eine Schnittstelle mit den Merkmalen des Anspruchs 1 bzw. mit einer Vorrichtung mit den Merkmalen des Anspruchs 13 Rechnung getragen.

Erfindungsgemäß kann der Köcher nach wie vor getrennt vom beweglichen Element der Schnittstelle, nämlich von der Frontplatte eines Dialysegeräts montiert sein. Somit kann eine aufwendige Integration eines feststehenden Elements in die Frontplatte, was wegen der erforderlichen Abdichtungen viele schlecht zu reinigende Stellen nach sich ziehen würde, entfallen. Ferner wird eine Zentrierflächen-, beispielsweise eine Konusflächenpaarung oder Kugel-/Kalottenflächenpaarung, zwischen in der Vorrichtung montiertem Aufnahmerohr eines Köchers für einen Ansaugstab des Dialysegeräts, und der Frontplatte, vorgesehen, mit dem Vorteil, dass sich über diese Flächen eine zentrierende großflächige Anlage der Schnittstellen-Elemente erzielen lässt. Die Anordnung ist ferner derart getroffen, dass bei der Herstellung einer vorzugsweise abgedichteten Fügung der Schnittstelle das in der Vorrichtung montierte Element, also der Köcher bei der Bewegung der Frontplatte, beispielsweise einer Schwenkbewegung, und der damit einhergehenden Annäherung der Zentrierflächen mit sich erst langsam in Fluchtung kommenden Achsen in axialer Richtung gegen die Kraft einer Vorspannfeder bewegbar ist, während seine führende Lagerung in der Vorrichtung eine Bewegung zulässt, die es erlaubt, die Zentrierflächen zwängungsfrei in Fügeposition mit zueinander fluchtenden Zentrierflächenachsen zu bringen. Bedingt durch das federbelastete Element, welches auch einen anfänglichen Winkelversatz während des Bewegungsmusters, beispielsweise des Verschwenkens der Frontplatte zulässt, werden die zu fügenden Bauteile passend in der Schnittstelle positioniert, wobei jegliche Toleranzen ausgeglichen werden. Die Schnittstelle wird somit allein durch die Bewegung der Frontplatte gefügt. Zeitraubende Justierungen an der Schnittstelle können entfallen.

Darüber hinaus ergibt sich der zusätzliche Vorteil, dass die Schnittstelle an beliebiger Stelle an der Front der Vorrichtung positioniert werden kann, und dass bei der Ausbildung mehrerer Schnittstellen die Lagezuordnung dieser Schnittstellen zueinander frei wählbar sind. Dadurch können beispielsweise zwei Spülkammern (Köcher) nebeneinanderliegend positioniert werden, um vertikalen Bauraum einzusparen.

Vorteilhafte Weiterbildungen sind Gegenstand der Unteransprüche.

Es zeigt sich, dass es mittels eines einfachen Gleitlagers für das rohrförmige, in der Vorrichtung montierte Element, also für den Köcher, gelingt, eine saubere und dauerhaft haltbare Positionierung des Köchers sicherzustellen, zumal die beim Fügen der Schnittstelle auftretende axiale Bewegung des Köchers im mm-Bereich liegt.

Der beim Fügen der Schnittstelle zum Ausgleich des Winkelversatzes der Zentrierflächen erforderliche Freiheitsgrad der Lagerung lässt sich auf verschiedenste Weise realisieren. Eine vorrichtungstechnisch einfache Variante besteht vorteilhafterweise darin, das Gleitlager in einem Lagerblock auszubilden, der in der Vorrichtung zumindest in einer Ebene beweglich ist, die senkrecht auf einer Bewegungsachse der Frontplatte steht. Für die benötigte seitliche Beweglichkeit genügt es, eine leicht spielbehaftete Befestigung des Lagerblocks in der Vorrichtung vorzusehen.

Wenn das Gleitlager über eine Kugelkalotte in einem Lagerblock fixiert ist, wird dem rohrförmigen Element, also dem Aufnahmerohr des Köchers eine erweiterte Bewegungsfreiheit gegeben, während gleichzeitig eine exakte axiale Führung aufrecht erhalten bleibt. Lagerungen, bei denen ein Gleitlager über eine Kugelkalotte in einem Lagerblock fixiert ist, stehen als Bauelemente zur Verfügung, beispielsweise als sogenannte Stehlager, die unter der Bezeichnung igubal^{®} mit iglidur^{®} W300 Kalotte im Handel sind.

Eine vorrichtungstechnisch besonders einfache Lagerung des Köchers wird geschaffen, wenn das Gleitlager eine leicht konische Lagerfläche hat, die sich beispielsweise zum stirnseitigen Ende des Köchers hin verjüngt. Der Konuswinkel kann dabei im kleinen einstelligen Gradbereich liegen. Diese Gestaltung hat den zusätzlichen Vorteil, dass der Konuswinkel dann, wenn die Lagerbuchse als Spritzgussteil ausgebildet wird, ohnehin als Entformungsschräge erwünscht ist.

Wenn sich die Vorspannfeder am Lagerblock abstützt, kann die Druckfeder mit einer verhältnismäßig großen Länge ausgebildet werden, wodurch die Federkennlinie genauer festgelegt und in hinsichtlich der auftretenden Kontaktkräfte optimale Bereiche gelegt werden kann.

Grundsätzlich ist es möglich, die außenliegenden Zentrierflächen entweder auf dem Köcher oder auf dem beweglichen Element auszubilden. Fertigungstechnisch sinnvoller ist es jedoch, wenn eine Außenzentrierfläche an der Stirnseite des Köchers ausgebildet ist, die in zentrierenden Fügepassungseingriff mit einer Innenzentrierfläche in der Frontplatte bringbar ist.

Wenn am Köcher die Außenkonusfläche an einen Radialbund anschließt, in dem eine Ringdichtung zur Anlage an einer Innenwandung der Frontplatte aufgenommen ist, ergibt sich beim Fügen der Schnittstelle eine sichere Flüssigkeitsabdichtung zum Innenraum der Vorrichtung. Dabei kann durch geeignete Auswahl der Ringdichtung ohne Weiteres dafür Sorge getragen werden, dass die elastische Verformung der Ringdichtung einen vollständigen Zentrierflächenkontakt zulässt, so dass jegliche Spaltbildung im gefügten Zustand der Schnittstelle vermieden ist.

Eine weitere Vereinfachung der Gestaltung des stirnseitigen Endes des Köchers ergibt sich mit der Variante, gemäß der in der Außenzentrierfläche eine umlaufende Rille zur Aufnahme des Dichtungsrings ausgebildet ist.

Wenn die Achse des Köchers des Dialysegeräts zu einer horizontalen Ebene von der Schnittstelle nach unten abfallend unter einem Winkel geneigt ist, der beispielsweise im Bereich zwischen 20° und 30° liegt, sind die Öffnungen des Köchers für die Bedienungsperson des Dialysegeräts leichter zugänglich, vor allem auch dann, wenn die Schnittstelle am unteren Ende einer Frontplatte des Dialysegeräts angeordnet ist. Es hat sich gezeigt, dass sich mit der erfindungsgemäßen Ausgestaltung sogar Neigungswinkel bis zu 60°, vorzugsweise bis zu 45°, besonders bevorzugt bis zu 30° ohne Weiteres realisieren lassen.

Die Bedienungsfreundlichkeit wird gleichermaßen dann verbessert, wenn die Zentrierfläche in der Frontplatte eine Achse hat, die um einen Anstellwinkel von einigen Winkelgraden zu einer auf der Bewegungsachse der Frontplatte senkrechten Ebene geneigt ist. Der vorstehend beschriebene Neigungswinkel der Köcherachse lässt sich damit leichter vergrößern.

Herstellungstechnisch vereinfacht wird die Schnittstelle, wenn die Zentrierflächenpaarung von einer Konusflächenpaarung gebildet ist.

Ein besonders vorteilhaftes Anwendungsgebiet der Schnittstelle stellt eine Vorrichtung zur extrakorporalen Blutbehandlung, insbesondere Dialysemaschine, mit zumindest einer Schnittstelle wie voranstehend beschrieben dar. Dabei ist das bewegliche Element der Schnittstelle eine über eine Scharnierverbindung an einem Gestell angebrachte Frontplatte, die vorzugsweise einen Einsatz aufnimmt, in dem die Zentrier- bzw. Konusfläche ausgebildet ist.

Die Schnittstelle hat den besonderen Vorteil, dass sie für mehrere vorzugsweise horizontal oder vertikal nebeneinander liegende Zugänge zur Vorrichtung, also beispielsweise für mehrere Steckplätze von Saugrohren eines Dialysegeräts, in gleicher Weise ausgebildet sein kann. Es können also identisch ausgebildete Schnittstellen vorliegen, obwohl in diesem Fall die Bewegungskurven der Zentrierflächen am beweglichen Element durch den unterschiedlichen Abstand zur Scharnierachse der Frontplatte unterschiedlich große Radien haben. Die Zugänge müssen dabei nicht horizontal nebeneinander liegen, sie können auch vertikal übereinander positioniert sein, wenn die Frontplatte ein Scharniergelenk mit horizontaler Achse hat.

In diesem Fall ist es sogar möglich, den nebeneinanderliegend montierten rohrförmigen Elementen einen gemeinsamen Lagerblock zuzuweisen, wodurch die Konstruktion weiter vereinfacht wird.

Nachstehend werden Ausführungsbeispiele der Erfindung anhand schematischer Zeichnungen näher erläutert. Es zeigen:
Figur 1 eine perspektivische Teildarstellung eines Dialysegeräts mit einem Zugang für zwei Saugstäbe zu einem Köcher;
Figur 2 in vergrößertem Maßstab die Einzelheit II in Figur 1 ;
Figur 3 eine perspektivische Ansicht zweier horizontal nebeneinander angeordneter Köcher des Dialysegeräts;
Figur 4 eine vergrößerte perspektivische Ansicht der in Figur 3 gezeigten Köcher im Zusammenwirken mit einem Einsatz in einer Frontplatte des Dialysegeräts;
Figur 5 in vergrößerter Darstellung eine perspektivische Ansicht des in Figur 4 gezeigten Einsatzes in der Frontplatte;
Figur 6 eine perspektivische Ansicht der Stirnseite des axial beweglich geführten rohrförmigen Elements der Schnittstelle;
Figuren 7A und 7B schematische Schnittdarstellungen der Frontplatte und des Köchers zu verschiedenen Zeitpunkten des Fügevorgangs der Schnittstelle zwischen Köcher und Frontplatte;
Figur 8A und 8B perspektivische Ansichten der führenden Lagerung des rohrförmigen Elements bei geöffneter und geschlossener Frontklappe des Dialysegeräts;
Figur 9 in vergrößerter Darstellung die Seitenansicht der Lagerung für das rohrförmige Element;
Figur 10 eine perspektivische Teil-Durchscheinungsansicht einer Seite eines Lagerblocks für die Lagerung gemäß Figur 9;
Figur 11 eine der Figur 8 ähnliche perspektivische Ansicht einer Variante der Lagerung für das rohrförmige Element;
Figur 12 eine perspektivische Ansicht des in Figur 11 gezeigten Lagerblocks;
Figur 13 eine Schnittansicht einer modifizierten Ausführungsform des Köchers mit Lagerung im Dialysegerät;
Figur 14 eine perspektivische Ansicht zweier nebeneinander im Dialysegerät montierter Köcher in der Ausgestaltung gemäß Figur 13; und
Figur 15 in etwas vergrößertem Maßstab die perspektivische Schnittansicht der von Frontplatte und Köcher gemäß Figur 13 bzw. 14 gebildeten Schnittstelle im gefügten Zustand.

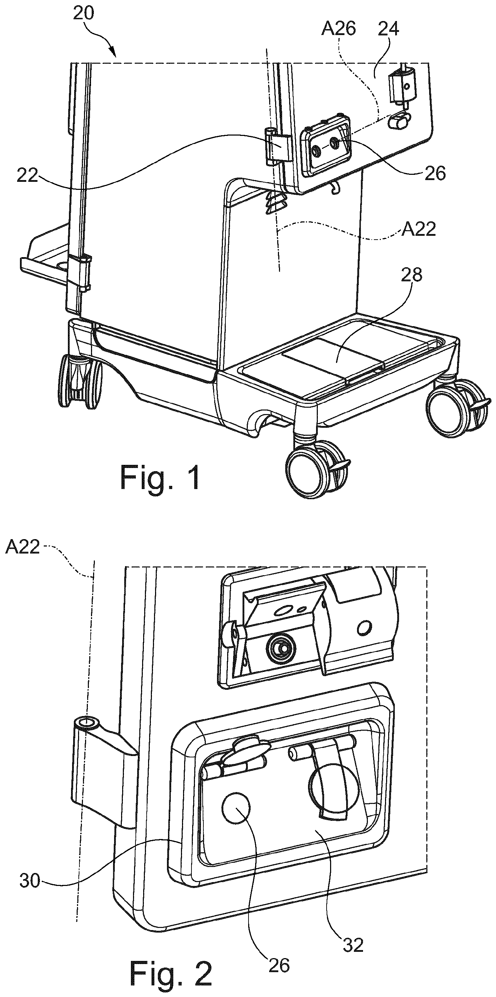

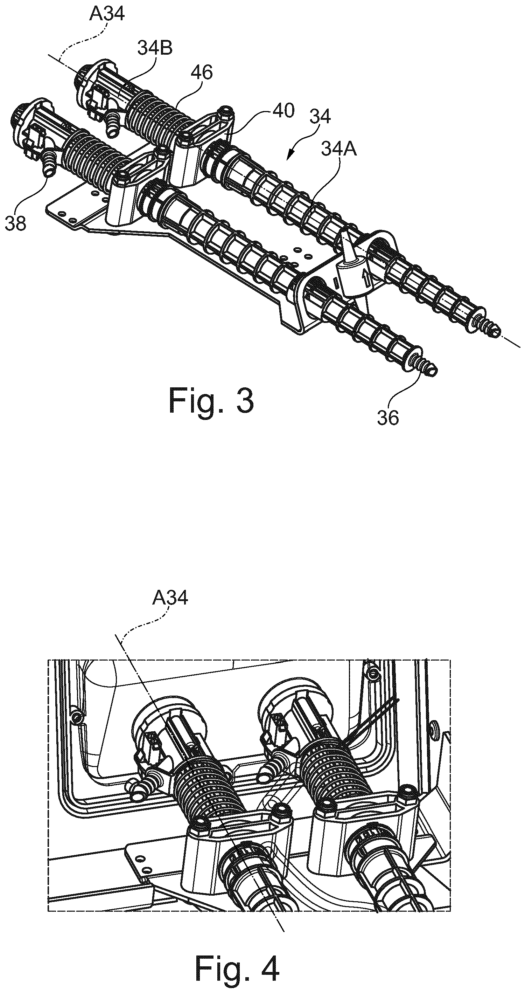

In Figur 1 ist mit dem Bezugszeichen 20 ein Dialysegerät bezeichnet, an dem über eine Scharnierverbindung 22 mit der Scharnierachse A22 eine Frontplatte 24 angelenkt ist. In die Frontplatte 24 eines Dialysegeräts sind regelmäßig verschiedenste Aggregate und Steckplätze integriert, wie z.B. Peristaltikpumpen, Infusoren und dgl., sowie Zugänge 26 zu sogenannten - in Figur 3 gezeigten - Köchern, in die beispielsweise Ansaugstäbe gesteckt werden können, die über Schläuche an nicht dargestellte Kanister angeschlossen sind, welche beispielsweise auf einer Bodenplatte/Sockel 28 des Dialysegeräts 20 unterhalb der Frontplatte 24 positioniert sind. Die Köcher 34 sind im gezeigten Ausführungsbeispiel identisch ausgebildet, sie können jedoch auch unterschiedlich dimensioniert sein.

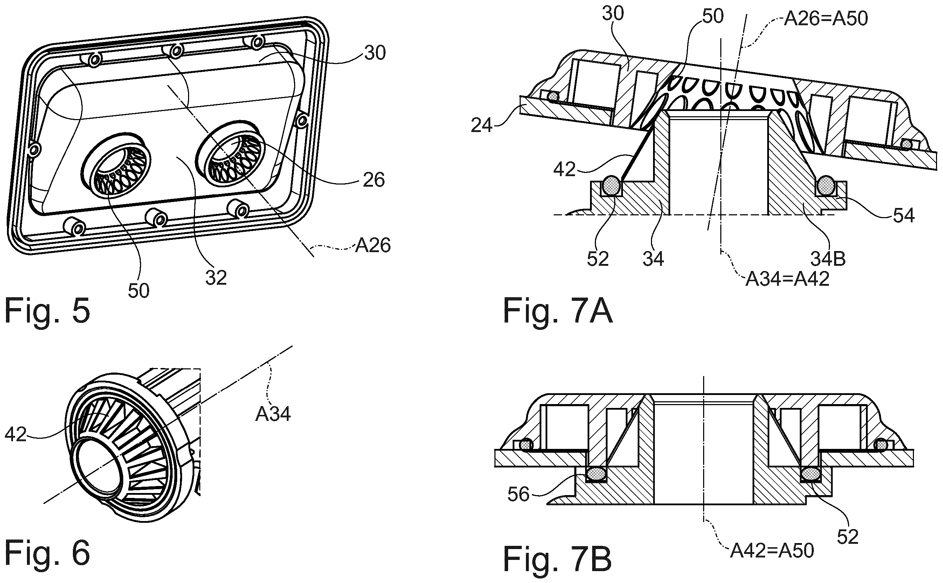

Die Zugänge 26 sind vergrößert in Figur 2 gezeigt. Sie sitzen in einem Einsatz 30, der in Figur 5 in vergrößerter Darstellung gezeigt ist und eine zur Frontplatte 24 um einige Grade geneigte Einsatzwand 32 hat, so dass die Zugänge 26 von der Bedienungsperson des Dialysegeräts leichter zugänglich sind und von oben besser gesehen werden können. Die Achsen der in der Regel senkrecht auf der Einsatzwand 32 stehenden Achsen der Zugänge 26 sind - wie am besten aus der Figur 5 ersichtlich - mit A26 bezeichnet.

Bei geschlossener Frontklappe 24 liegen die Zugänge 26 - wie in Figur 4 dargestellt - axial und radial fluchtend und abgedichtet vor den Köchern 34, die eine Achse A34 haben und im Dialysegerät 20 in der Regel so montiert sind, dass die Achse A34 bezüglich einer Horizontalebene um einen bestimmten kleinen Winkel geneigt ist, der bis in Bereiche von 60°, vorzugsweise bis 45° gelegt werden kann, und beispielsweise zwischen 20° und 30° liegt. Hierzu wird eine in Folgenden näher zu beschreibende zentrierende Schnittstelle zwischen Köcher 34 und Frontplatte 24 geschaffen, die es erlaubt, den in dem Dialysegerät 20 montierten Köcher 34 beim Schließen der Frontplatte 24 sauber und spaltfrei abzudichten, ohne dass aufwendige Montage- und Justierhandgriffe erforderlich werden.

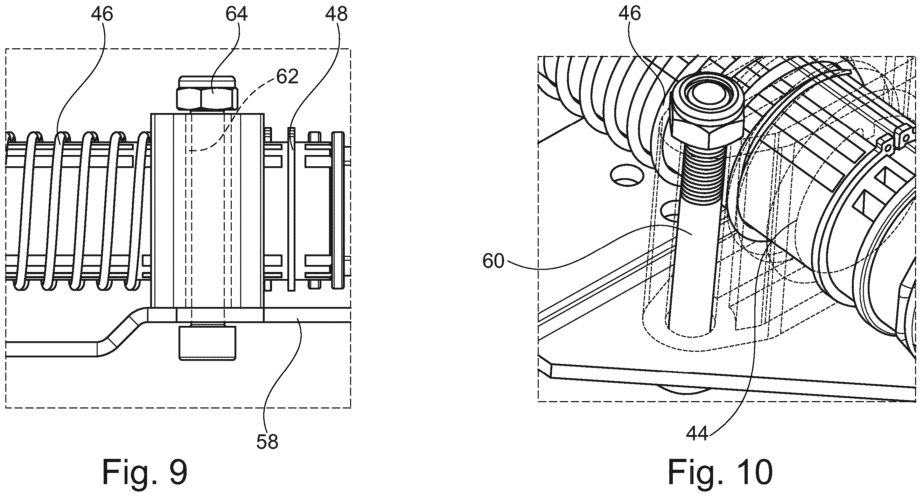

Die Köcher 34 weisen ein im Dialysegerät 20 verbautes rohrförmiges inneres Bauteil 34A mit einem Zulaufanschluss 36, beispielsweise für ein Desinfektionsmittel, und ein dazu koaxiales äußeres rohrförmiges Bauteil 34B auf, das mit Ablaufanschlüssen 38 und stirnseitig mit einer zentrierenden Fügefläche 42 - siehe Figur 6 - ausgestattet ist. Im gezeigten Ausführungsbeispiel ist die Fügefläche 42, die im Folgenden als köcherseitige Zentrierfläche bezeichnet wird, von einer über den Umfang mehrfach unterbrochenen Konusfläche gebildet, so dass die Achse A42 der Zentrierfläche 42 mit der Achse A34 zusammenfällt. Der Köcher 34 ist axial begrenzt beweglich in einem Lagerblock 40 geführt, der eine Gleitlagerung für den Köcher 34 ausbildet. Genauer gesagt, ist der Köcher 34 in einer zylindrischen Lagerbohrung 44 (siehe Figur 10) des Lagerblocks 40 aufgenommen, an dem sich eine Vorspannfeder 46 abstützt, die den Köcher 34 und damit das rohrförmige Bauteil 34B in Richtung Frontplatte 24 drückt.

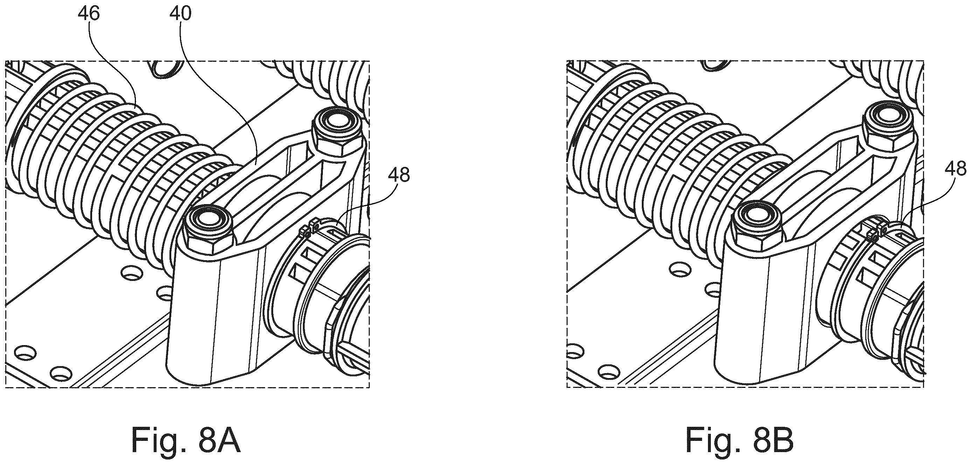

Im nicht gefügten Zustand der Schnittstelle, also bei geöffneter Frontplatte 24 drückt die Vorspannfeder 46 den Köcher 34 in die in Figur 8A gezeigte Anschlagstellung, in der ein Axialsicherungsring 48 am Lagerblock 40 anschlägt.

Als Gegenstück zur ersten Zentrierfläche 42 am Köcher 34 ist in der Einsatzwand 32 eine frontklappenseitige Zentrierfläche 50 in der Ausgestaltung als Konusfläche mit einer mit der Achse A26 zusammenfallenden Achse A50 derart ausgebildet, dass die Zentrierflächen 42 und 50 eine Zentrierflächenpaarung bilden, die beim Schließen der Frontplatte 24 dafür sorgt, dass die Öffnung des Köchers 34 abgedichtet in der Frontplatte 24 positioniert ist. Hierzu ist Folgende Anordnung getroffen:
Aufgrund der beweglichen Anbringung der Frontplatte 24 am Dialysegerät 20 beschreibt die Zentrierfläche 50 in der Einsatzwand 32 eine Kreisabschnittsbewegung, so dass die Achse A26 der Zentrierfläche 50 zu Beginn der Schließbewegung der Frontplatte 24 mit der Achse A34 der Zentrierfläche 42 am Köcher 34 nicht fluchtet. Dieser Zustand ist in Figur 7A schematisch dargestellt.

Mit dem weiteren Verlauf des Schließvorgangs nähern sich die Zentrierflächen 42, 50 immer mehr, so dass die Achsen A26 und A34 allmählich in Fluchtung zueinander gelangen. Durch den anfänglichen exzentrischen Kontakt der Zentrierflächen 42 und 50 wird das Bauteil 34B des Köchers 34 gegen die Kraft der Vorspannfeder 46 axial verschoben. Gleichzeitig ist die Anordnung so getroffen, dass die das Bauteil 34B führende Lagerung eine Ausgleichsbewegung zulässt, die es erlaubt, die Zentrierflächen 42, 50 zwängungsfrei in die in Figur 7B dargestellte Fügeposition mit zueinander fluchtenden Zentrierflächenachsen A42 und A50 zu bringen. In dieser Fügeposition liegen die als Konusflächen ausgestalteten Zentrierflächen 42, 50 satt aneinander an, so dass eine vollkommene Zentrierung der Schnittstelle gegeben ist. In dieser Position, die in Figur 8B dargestellt ist, in der der Axialsicherungsring 48 vom Lagerblock 40 abgehoben ist, wird ein in einer Ringnut 52 eines Radialbunds 54 aufgenommener Dichtungsring 56 elastisch von der Frontplatte 24, genauer gesagt von dem in der Frontplatte 24 sitzenden Einsatz 30 (siehe Figur 7B) verformt, wodurch der Zugang 26 des Steckplatzes fluiddicht abgedichtet wird.

Die Achsen A26 und A34 können vereinfacht in Fluchtung gebracht werden, wenn die Zentrierfläche 50 in der Frontplatte 24 eine Achse A50 hat, die bezüglich einer auf der Scharnierachse A22 der Frontplatte 24 senkrecht stehenden Ebene um einen vorbestimmten Winkel zum Dialysegerät 20 nach unten abfallend geneigt ist.

Da die geführte Lagerung des Bauteils 34B des Köchers 34 ein zylindrisches Gleitlager ist, und weil die Bewegung der Frontplatte 24 beim Schließvorgang einer Kreisbewegungskurve folgt, ist der Lagerblock 40 wie folgt im Dialysegerät 20 montiert:
Figur 9 und 10 lassen erkennen, dass der Lagerblock 40 auf einer Platte 58 montiert ist, die in einer Ebene liegt, welche senkrecht auf der Achse A22 der Scharniere 22 der Frontplatte 24 steht. Dies ist gleichbedeutend damit, dass mit 60 bezeichnete Befestigungsbozen des Lagerblocks 40 parallel zur Scharnierachse A22 verlaufen. Damit beim Schließen und Öffnen der Frontplatte der Winkelversatz bzw. der Fluchtungsfehler ausgeglichen werden kann, wird ein vorbestimmtes Spiel in der Verschraubung des Lagerblocks 40 ausgenutzt. Dazu werden die zur Aufnahme der Befestigungsbozen 60 im Lagerblock 40 vorgesehenen Bohrungen 62 (siehe Figur 9) im Durchmesser um ein vorbestimmtes Aufmaß größer hergestellt als der Bolzendurchmesser. Eine selbstsichernde Mutter 64 wird nach einem anfänglichen Festziehen zur Verschraubung des Lagerblocks 40 leicht gelockert, wodurch dem Lagerblock 40 das erforderliche Bewegungsspiel zum Ausgleich des vorstehend beschriebenen Winkelversatzes gegeben wird.

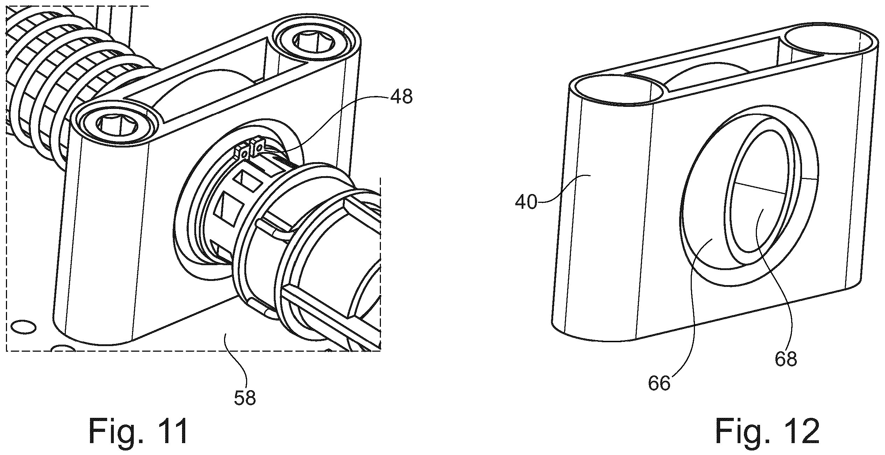

Bei dem oben beschriebenen Ausführungsbeispiel ist das Bauteil 34B in einem zylindrischen Gleitlager geführt, welchem eine lineare Verschiebebewegung erlaubt wird. Anhand der Figuren 11 und 12 wird eine modifizierte Art der Führung des Bauteils 34B beschrieben.

Der fest auf der Platte 58 verschraubte Lagerblock 40 nimmt bei dieser Variante ein Gleitlagerteil 66 in der Ausgestaltung als Kugelkalotte auf, das eine zylindrische Lagerbohrung 68 zur Aufnahme des Bauteils 34B des Köchers 34 hat. Derartige Lager sind als sogenannte Stehlager auf dem Markt verfügbar und werden beispielsweise von der Firma IGUS GmbH unter der Bezeichnung igubal^{®} Stehlager mit iglidur^{®} W300 Kalotte vertrieben.

Diese Art der Lagerung gibt dem Köcher 34 eine zusätzliche Bewegungsfreiheit hinsichtlich der Ausrichtung der Achse A34 des Köchers 34, so dass es zur Kompensation des Winkelversatzes der Achsen A34 und A26 beim Schließen der Frontplatte 24 nicht mehr erforderlich ist, die Platte 58 senkrecht zur Scharnierachse A22 anzuordnen. Somit verbleibt mehr Spielraum für die Neigung der Achse des Köchers 34 im Dialysegerät 20.

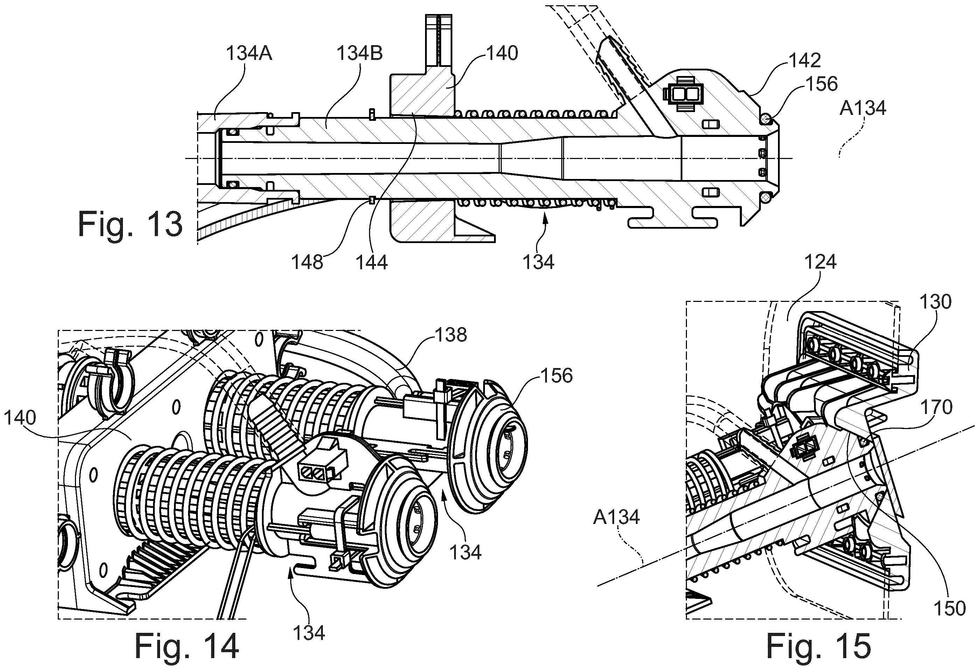

Nachstehend wir anhand der Figuren 13 bis 15 ein weiter abgewandeltes Ausführungsbeispiel der Schnittstelle beschrieben. Komponenten dieser Variante, die bestimmten Bauteilen der vorstehend beschriebenen Ausführungsbeispiele entsprechen, sind dabei mit entsprechenden Bezugszeichen versehen, denen eine "1" vorangestellt ist.

Der Figur 14 ist entnehmbar, dass zwei horizontal nebeneinander liegende Köcher 134 in einem gemeinsamen Lagerblock 140 geführt gelagert sind.

Der mit 134 bezeichnete Köcher ist - wie der Längsschnitt durch den Köcher 134 gemäß Figur 13 zeigt - so aufgebaut, dass ein vorderes Bauteil 134B, das wieder stirnseitig eine konische Zentrierfläche 142 ausbildet, die über den Umfang mehrfach unterbrochen ist, gleitend verschiebbar in einem ortsfest im Dialysegerät 20 montierten Bauteil 134A aufgenommen ist. Die komplementäre konische Zentrierfläche im Einsatz 130, der in der Frontplatte 124 sitzt, ist mit dem Bezugszeichen 150 bezeichnet. Abweichend vom Ausführungsbeispiel der Figuren 1 bis 12 ist die geführte Lagerung des Bauteils 134B im Lagerblock 140 so ausgebildet, dass sie von einer leicht konischen Lagerfläche 144 gebildet wird. Diese Lagerfläche kann einen Konuswinkel von einigen wenigen Winkelgraden haben und sie kann sich in beliebiger Richtung erweitern.

Im gezeigten Ausführungsbeispiel erweitert sich die konische Lagerfläche zum in der Vorrichtung montierten Bauteil 134A. Mit dieser Gestaltung der Lagerung des Köchers 314 wird diesem beim Schließen der Frontplatte 24 der erforderliche Bewegungsfreiheitsgrad gegeben, damit die Zentrierflächen 142, 150 zwängungsfrei in die in Figur 15 gezeigte Fügeposition mit zueinander fluchtenden Konusflächenachsen gebracht werden können, während das Bauteil 134B des Köchers 134 gegen die Kraft der Vorspannfeder 146 nach innen, d.h. auf den Lagerblock 140 zu, verschoben wird. Über die Wahl des Konuswinkels der Lagerfläche 144 kann Einfluss auf die zulässige Neigung der Längsachse A134 des Köchers 134 genommen werden. Auf diese Weise kann beispielsweise die Achse A134 des Köchers 134 zu einer horizontalen Ebene um einen Winkel geneigt werden, der im Bereich zwischen 20° und 30° liegt.

In weiterer Abweichung vom oben beschriebenen Ausführungsbeispiel ist der mit dem Bezugszeichen156 bezeichnete Dichtungsring in einer umlaufenden Rille 170 aufgenommen, die in der als Außenkonus gestalteten Zentrierfläche 142 des Bauteils 134B ausgebildet ist. In der in Figur 15 gezeigten Fügestellung der Schnittstelle ist der Dichtungsring 156 elastisch so weit zusammengedrückt, dass die Zentrierflächen 142, 150 der Zentrierflächenpaarung vollflächig aneinander anliegen, so dass ihre jeweiligen Achsen zusammenfallen.

Selbstverständlich sind Abweichungen vom beschriebenen Ausführungsbeispiel möglich, ohne den Grundgedanken der Erfindung zu verlassen.

Die Anzahl und Lagezuordnung der Schnittstellen können frei variiert werden, ohne das Grundprinzip der justierfreien Abdichtung abändern zu müssen.

Vorstehend sind die Zentrierflächen als Konusflächen beschreiben worden. Sie können jedoch auch von Kugelsegment- und Kugelkalottenflächen usw. gebildet werden.

Die Ausführungsbeispiele haben einen Aufbau, bei denen die konvexe Zentrierfläche am Köcher ausgebildet ist, während die konkave Zentrierfläche im Einsatz der Frontplatte vorliegt. Diese Anordnung kann jedoch auch umgekehrt werden.

Die Bewegung der Frontplatte ist auch nicht auf eine Schwenkbewegung um eine Scharnierachse beschränkt. Die Schnittstelle kann auch bei anderen Bewegungsmustern des beweglichen Elements, beispielsweise der Frontplatte eines Dialysegeräts gleichermaßen zum Einsatz kommen.

Die Erfindung schafft somit eine zentrierende Schnittstelle zwischen einem in einer Vorrichtung montierten rohrförmigen Element, insbesondere einem Aufnahmerohr (Köcher) für einen Ansaugstab eines Dialysegeräts, und einem beispielsweise schwenkbar um eine Achse geführten beweglichen Element, insbesondere einer Frontplatte des Dialysegeräts. Es ist eine Konusflächenpaarung zwischen in der Vorrichtung montiertem Element und beweglichem Element vorgesehen, und bei der Herstellung einer vorzugsweise abgedichteten Fügung der Schnittstelle ist das in der Vorrichtung montierte Element bei der Bewegung des beweglichen Elements und der damit einhergehenden Annäherung der Konusflächen in axialer Richtung gegen die Kraft einer Vorspannfeder bewegbar, während seine führende Lagerung eine Bewegung zulässt, die es erlaubt, die Zentrierflächen zwängungsfrei in Fügeposition mit zueinander fluchtenden Zentrierflächenachsen zu bringen.

**Bezugszeichenliste**

| | | | |
|---|---|---|---|
| 20 | Dialysegerät | | |
| 22 | Scharnierverbindung | A22 | Scharnierachse |
| 24, 124 | Frontplatte | | |
| 26 | Zugänge | A26 | Zugangsachse |
| 28 | Bodenplatte | | |
| 30, 130 | Einsatz | | |
| 32 | Einsatzwand | | |
| 34, 134 | Köcher | A34, A134 | Köcherachse |
| 36 | Zulaufanschluss | | |
| 38, 138 | Ablaufanschluss | | |
| 40, 140 | Lagerblock | | |
| 42, 142 | Zentrierfläche | A42 | Zentrierflächenachse |
| 44 | Lagerbohrung | | |
| 46 | Vorspannfeder | | |
| 48, 148 | Axialsicherungsring | | |
| 50, 150 | Zentrierfläche | A50 | Zentrierflächenachse |
| 52 | Ringnut | | |
| 54 | Radialbund | | |
| 56, 156 | Dichtungsring | | |
| 58 | Platte | | |
| 60 | Befestigungsbozen | | |
| 62 | Bohrungen | | |
| 64 | Mutter | | |
| 66 | Gleitlagerteil | | |
| 68 | Lagerbohrung | | |
| 144 | konische Lagerfläche | | |
| 170 | Rille | | |

## Patentansprüche

1. Zentrierende Schnittstelle zwischen einem in einer Vorrichtung montierten Aufnahmerohr eines Köchers (34; 134) für einen Ansaugstab eines Dialysegeräts (20) und einer beweglichen, vorzugsweise schwenkbar um eine Achse (A22) geführten, Frontplatte (24; 124) des Dialysegeräts (20), bei dem
eine Zentrierflächenpaarung (42, 50) zwischen dem Köcher (34; 134) und der Frontplatte vorgesehen ist, und
bei der Herstellung einer vorzugsweise abgedichteten Fügung der Schnittstelle der Köcher (34; 134) bei der Bewegung der Frontplatte (24; 124) und der damit einhergehenden Annäherung der Zentrierflächen (42, 50; 142, 150) in axialer Richtung gegen die Kraft einer Vorspannfeder (46) bewegbar ist, während seine führende Lagerung eine Bewegung zulässt, die es erlaubt, die Zentrierflächen (42, 50) zwängungsfrei in Fügeposition mit zueinander fluchtenden Zentrierflächenachsen (A42, A50) zu bringen.

2. Schnittstelle nach Anspruch 1, **dadurch gekennzeichnet, dass** der Köcher in einem Gleitlager geführt ist.

3. Schnittstelle nach Anspruch 2, **dadurch gekennzeichnet, dass** die Frontplatte (24) schwenkbar um eine Achse (A22) geführt ist, und das Gleitlager in einem Lagerblock (40) sitzt, der in der Vorrichtung zumindest in einer Ebene beweglich ist, die senkrecht auf der Bewegungsachse (A22) der Frontplatte steht.

4. Schnittstelle nach Anspruch 2, **dadurch gekennzeichnet, dass** das Gleitlager über eine Kugelkalotte in einem Lagerblock (40) fixiert ist.

5. Schnittstelle nach einem der Ansprüche 2 bis 4, **dadurch gekennzeichnet, dass** das Gleitlager eine leicht konische Lagerfläche (144) hat, die sich zu einem Ende des Köchers (134) hin erweitert.

6. Schnittstelle nach einem der Ansprüche 3 bis 5, **dadurch gekennzeichnet, dass** sich die Vorspannfeder (46; 146) am Lagerblock (40; 140) abstützt.

7. Schnittstelle nach einem der Ansprüche 1 bis 6, **dadurch gekennzeichnet, dass** an der Stirnseite des Köchers (34) als Zentrierfläche (42) eine Außenkonusfläche ausgebildet ist, die in zentrierendem Fügepassungseingriff mit einer als Zentrierfläche (50) dienenden Innenkonusfläche in der Frontplatte (24), bringbar ist.

8. Schnittstelle nach Anspruch 7, **dadurch gekennzeichnet, dass** am Köcher (34) die Außenzentrierfläche (42) an einen Radialbund (54) anschließt, in dem eine Ringdichtung (56) zur Anlage an einer Innenwandung der Frontplatte (24) aufgenommen ist.

9. Schnittstelle nach Anspruch 7, **dadurch gekennzeichnet, dass** in der Außenzentrierfläche (142) eine umlaufende Rille (170) zur Aufnahme eines Dichtungsrings (156) ausgebildet ist.

10. Schnittstelle nach einem der Ansprüche 1 bis 9, **dadurch gekennzeichnet, dass** eine Achse (A34) des Köchers (34) zu einer horizontalen Ebene unter einem Winkel bis zu 60°, vorzugsweise bis zu 45°, besonders bevorzugt bis zu 30° geneigt ist.

11. Schnittstelle nach einem der Ansprüche 1 bis 10, **dadurch gekennzeichnet, dass** die Zentrierfläche (50) in der Frontplatte (24) eine Achse (A50) hat, die bezüglich einer auf einer Bewegungsachse (A22) der Frontplatte senkrechten Ebene um einen vorzugsweise spitzen Winkel angestellt ist.

12. Schnittstelle nach einem der Ansprüche 1 bis 11, **dadurch gekennzeichnet, dass** die Zentrierflächenpaarung (42, 50; 142, 150) von einer Konusflächenpaarung gebildet ist.

13. Vorrichtung zur extrakorporalen Blutbehandlung, insbesondere Dialysegerät, mit zumindest einer Schnittstelle nach einem der Ansprüche 1 bis 12.

14. Vorrichtung nach Anspruch 13, bei der eine über eine Scharnierverbindung an einem Gehäuse des Dialysegeräts angebrachte Frontplatte (24; 124) einen Einsatz (30; 130) aufnimmt, in dem die Zentrierfläche (50; 150) ausgebildet ist.

15. Vorrichtung nach Anspruch 14, **dadurch gekennzeichnet, dass** der Einsatz (30; 130) zumindest zwei in einer zur Bewegungsachse (A22) der Frontplatte senkrechten Ebene, vorzugsweise horizontal oder vertikal nebeneinanderliegende Zentrierflächen (150) für die entsprechend nebeneinanderliegend montierten Köcher (134) ausbildet, wobei vorzugsweise den nebeneinanderliegend montierten Köchern (134) ein gemeinsamer Lagerblock (140) zugewiesen ist.
